(19) Europäisches Patentamt
European Patent Office
Office européen des brevets

(11) Veröffentlichungsnummer: **0 267 438 B1**

# (12) EUROPÄISCHE PATENTSCHRIFT

(45) Veröffentlichungstag der Patentschrift: **16.10.91**

(51) Int. Cl.5: **C07C 255/07**, C07C 253/00

(21) Anmeldenummer: **87114832.6**

(22) Anmeldetag: **10.10.87**

## (54) Verfahren zur Herstellung von ungesättigten Nitrilen.

(30) Priorität: **14.10.86 DE 3634914**

(43) Veröffentlichungstag der Anmeldung:
**18.05.88 Patentblatt 88/20**

(45) Bekanntmachung des Hinweises auf die Patenterteilung:
**16.10.91 Patentblatt 91/42**

(84) Benannte Vertragsstaaten:
**BE CH DE FR GB LI NL**

(56) Entgegenhaltungen:
**EP-A- 0 196 554**
**EP-A- 0 232 712**
**FR-A- 1 256 179**
**US-A- 4 011 278**
**US-A-34 427 95**

**CHEMISTRY AND INDUSTRY, 270 (1984)**

(73) Patentinhaber: **BASF Aktiengesellschaft**
**Carl-Bosch-Strasse 38**
**W-6700 Ludwigshafen(DE)**

(72) Erfinder: **Hoelderich, Wolfgang, Dr.**
**Mannheimer Strasse 18 c**
**W-6710 Frankenthal(DE)**
Erfinder: **Schwarzmann, Matthias, Dr.**
**Carl-Bosch-Strasse 54**
**W-6703 Limburgerhof(DE)**

EP 0 267 438 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft ein Verfahren zur Herstellung von ungesättigten Nitrilen durch Spaltung von Lactonen mit Ammoniak in Gegenwart von Zeolithen als Katalysatoren.

Es ist bekannt, Nitrile durch thermische Wasserabspaltung aus Säureamiden oder Carbonsäuren bzw. deren Derivate und Ammoniak in Gegenwart geeigneter Katalysatoren herzustellen. Als Katalysatoren werden z.B. Aluminiumoxid, Siliciumdioxid, Manganoxid, Thoriumoxid oder Graphitkatalysatoren verwendet. Zur Erzielung hoher Umsätze und Ausbeuten sind Temperaturen von 400 bis 500 °C erforderlich. Die bekannten Verfahren haben den Nachteil, daß die Katalysatoren schnell desaktivieren und ihre Selektivität einbüßen.

Auch ist bekannt, daß man Lactone in Gegenwart von Ammoniak in Hydroxycarbonsäureamide überführen kann, die ihrerseits unter Wasserabspaltung leicht und schnell in die entsprechenden Lactame übergehen.

Die Reaktion von epsilon-Caprolacton mit Ammoniak in der Gasphase an Aluminiumoxid führt zu 22 % Hex-1-en-6-nitril und 24 % epsilon-Hydroxycapronsäurenitril.

Mit Titandioxid als Katalysator wird bei dieser Reaktion 70 bis 80 % und mit Zinkoxid 50 bis 73 % epsilon-Hydroxycapronsäurenitril gefunden. Auch mit Ni-, Os-, Ir-, Rh- und Pt-enthaltenden Hydrierungs- und Dehydrierungskatalysatoren, gegebenenfalls auf Trägern wie $Al_2O_3$, wird epsilon-Hydroxycapronsäureamid erhalten. Werden mit Ammoniummolybdat getränkte Tonscherben eingesetzt, erhält man aus Lactonen Lactame und Hydroxycarbonsäureamide. Aus Lactonen und Ammoniak in Gegenwart von Wasserstoff werden an Aluminiumoxid Hydroxycarbonsäurenitrile bzw. Aminoalkanole erhalten (US-PS 3 560 550).

Aus der FR-A-1 256 179 ist ein Verfahren zur Herstellung von 5-Hexennitril aus ε-Caprolacton mit wasserfreiem Ammoniak in Gegenwart eines Dehydrierkatalysators aus aktivierter Tonerde bei 325 bis 550 °C im Dampfzustand bekannt.

Aus US-PS 2 375 005 ist ein zweistufiges Verfahren zur Herstellung von alpha, beta-ungesättigten Nitrilen aus Lactonen bekannt. Hierbei wird das Lacton mit einem großen Überschuß an $NH_3$ als Verdünnungsmittel bzw. Lösungsmittel in Gegenwart von Alkali in einem geschlossenen Behälter unter Druck zu beta-Hydroxycarbonsäureamid umgesetzt. Die hierfür notwendige Reaktionszeit beträgt mehrere Stunden. Dieses Amid wird dann in einer zweiten Stufe an Dehydratisierungskatalysatoren wie $Al_2O_3$ , $BaO_2$ $ThO_2$, $ZrO_2$, $AlPO_4$ umgesetzt.

Es war die Aufgabe gestellt, Lactone mit Ammoniak zu spalten und vollständig in einem Verfahrensschritt in die ungesättigten Nitrile überzuführen, ohne daß hierbei die Reaktion auf der Stufe der Hydroxycarbonsäureamide oder der Hydroxycarbonsäurenitrile stehen bleibt, und ohne daß es zur Lactam-Bildung kommt. Die eingesetzten Katalysatoren sollen sich durch hohe Aktivität und lange Standzeit auszeichnen.

Es wurde gefunden, daß man die genannten Vorteile bei der Herstellung von ungesättigten Nitrilen durch katalytische Spaltung von unsubstituierten oder Alkyl-, Aryl- oder Aralkyl-substituierten 5- bis 7-gliedrigen Lactonen mit $NH_3$ erzielt, wenn man die Umsetzung im Molverhältnis von Lacton zu Ammoniak von 1 : 1 bis 1 : 30 bei Drücken von 0,1 bis 100 bar in der Gasphase bei 100 bis 500 °C oder in der Flüssigphase bei 50 bis 220 °C in Gegenwart von Zeolithen als Katalysatoren durchführt.

Die Umsetzung wird vorteilhaft in der Gasphase bei einer Temperatur von 150 bis 500 °C ausgeführt.

Das neue Verfahren beruht auf einem überraschenden Effekt und ist insofern bemerkenswert, als die Lactonspaltung zu ungesättigten Nitrilen gegenüber der ebenfalls sauer katalysierten Lactambildung bevorzugt verläuft.

Das erfindungsgemäße Verfahren hat den Vorteil, daß man in einer Stufe, ausgehend von unsubstituierten oder Alkyl-, Aryl- oder Aralkyl-substituierten 5- bis 7-gliedrigen Lactonen zu ungesättigten Nitrilen gelangt.

Geeignete Lactone, Methylbutyrolactone und Valerolactone für das erfindungsgemäße Verfahren sind beispielsweise Caprolacton, 7-Methyl-caprolacton, 6-Ethyl-Valerolacton, 6-Methylvalerolacton, 5-Phenylbutyrolacton oder 3,5-Dimethylbutyrolacton. Bevorzugter Ausgangsstoff ist Caprolacton.

Die erfindungsgemäße Umsetzung läßt sich z.B. für den Fall von Caprolacton durch folgende Formelgleichung wiedergeben:

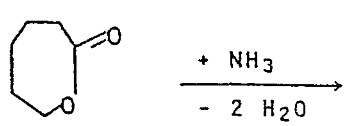


$CH_2 = CH - CH_2 - CH_2 - CH_2 - CN$

$CH_3 - CH = CH - CH_2 - CH_2 - CN$

$CH_3 - CH_2 - CH = CH - CH_2 - CN$

$CH_3 - CH_2 - CH_2 - CH = CH - CN$

Als Katalysatoren für das erfindungsgemäßen Verfahren verwendet man die Zeolithe zweckmäßig in der aciden Form. Zeolithe sind kristalline Aluminiumsilikate, die eine hochgeordnete Struktur mit einem starren dreidimensionalen Netzwerk von $SiO_4$- und $AlO_4$ Tetraedern besitzen, die durch gemeinsame Sauerstoffatome verbunden sind. Das Verhältnis der Si-und Al-Atome zu Sauerstoff beträgt 1 : 2. Die Elektrovalenz der Aluminium enthaltenden Tetraeder ist durch Einschluß von Kationen in den Kristall, z.B. eines Alkali- oder Wasserstoffions ausgeglichen. Ein Kationen-austausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordernit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man in Zeolithe vom Typ A, L, X oder Y.

Für das erfindungsgemäße Verfahren in Betracht kommende Katalysatoren sind Zeolithe aus der Mordernit-Gruppe oder engporige Zeolithe vom Erionit-bzw. Chabasit-Typ oder Zeolithe vom Faujasit-Typ, z.B. Y-, X- oder L-Zeolithe oder Zeolithe des Ferrierit-Typs.

In diese Gruppe von Zeoilithen gehören auch die sogenannten "ultrastabilen" Zeolithe des Faujasittyps, d.h. dealuminierte Zeolithe. Verfahren zur Herstellung solcher Zeolithe sind mannigfach beschrieben.

Besonders vorteilhaft sind Zeolithe vom Pentasiltyp. Diese haben als Grundbaustein einen aus $SiO_4$-Tetraedern aufgebauten Fünfring gemeinsam. Sie sind durch eine hohes $SiO_2/Al_2O_3$-Verhältnis gekennzeichnet sowie durch Porengrößen, die zwischen denen der Zeolithe vom Typ A und denen vom Typ X oder Y liegen.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithode oder deren Gemische sowie Alumino-, Boro, Gallium- und Eisengermanatzeolithe oder deren Gemische. Insbesondere eignen sich die Alumino-, Boro- und Eisensilikatzeolithe des Pentasiltyps für das erfindungsgemäße Verfahren. Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin-oder Triethylentetramin-Lösung mit oder insbesondere ohne Alkali- oder Erdalkalizusatz bei 100 bis 220 °C unter autogenem Druck hergestellt. Hierzu gehören auch die isotaktischen Zeolithe nach EP 34 727 und EP 46 504. Die erhaltenen Aluminosilikatzeolithe enthalten je nach Wahl der Einsatzstoffmengen ein $SiO_2/Al_2O_3$-Verhältnis von 10 bis 40 000. Diese Aluminosilikatzeolithe können in etherischem Medium wie Diethylenglykoldimethylether, in alkoholischem Medium wie Methanol bzw. 1,4-Butandiol oder in Wasser synthetisiert werden.

Borosilikatzeolithe kann man z.B. bei 90 bis 200 °C unter autogenem Druck synthetisieren, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Hierzu gehören auch die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Zu den erfindungsgemäß verwendbaren siliciumreichen Zeolithen ($SiO_2/Al_2O_3 \geqq 10$) gehören auch die verschiedenen ZSM-Typen, NU-1 und Silicalit ®.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200 °C unter autogenem Druck.

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90 : 10 bis 40 : 60 Gew. -% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Alumino-bzw., Borosilikatzeolith direkt

nach der Trocknung verformt wird und erst nach der Verformung einer Calcinierung unterworfen wird. Die hergestellten Alumino- und Borosilikatzeolithe können in reiner Form, ohne Binder, als Stränge oder Tabletten eingesetzt werden, wobei als Verstrangungs- oder Peptisierungshilfsmittel, z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohen Umsätzen sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetall wie Mg, Ca, Sr, Metalle der 3., 4. und 5. Hauptgruppe wie Al, Ga, Ge, Sn, Pb, Bi, Übergangsmetalle der 4. - 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Er, Yb, und U eingesetzt.

Zweckmäßigerweise führt man die Dotierung so durch, daß man den verformten Zeolithen in einem Steigrohr vorlegt und bei 20 bis 100 °C eine wäßrige oder ammonialkalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammonialkalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2$ x $H_2O$ oder $Ni(NO_3)_2$ x 6 $H_2O$ oder $Ce(NO_3)_3$ x 6 $H_2O$ oder $La(NO_3)_2$ x 6 $H_2O$ oder $Cs_2CO_3$ in Wasser löst und mit dieser Lösung den verformten oder unverformten Zeolith eine gewisse Zeit, z.B. 30 Minuten, tränkt. Die eventuell überstehende Lösung wird im Rotationsverdampfer von Wasser befreit. Danach wir der getränkte Zeolith bei etwa 150 °C getrocknet und bei etwa 550 °C calciniert. Dieser Tränkvorgang kann mehrmals hintereinander vorgenommen werden, um dem gewünschten Metallgehalt einzustellen.

Es ist auch möglich, eine wäßrige $Ni(NO_3)_2$-Lösung oder ammoniakalische $Pd(NO_3)_2$-Lösung herzustellen und darin den reinen pulverförmigen Zeolithen bei 40 bis 100 °C unter Rühren etwa 24 h aufzuschlämmen. Nach Abfiltrieren, Trocknen bei etwa 150 °C und Calcinierung bei etwa 500 °C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöhter Temperatur zwischen 30 und 80 °C im Kreislauf 15 bis 20 h leitet. Danach wird mit Wasser ausgewaschen, bei etwa 150 °C getrocknet und bei etwa 550 °C calciniert. Bei manchen metalldotierten Zeolithen, z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft, z.B. so vor, daß man Zeolithe in Pulverform mit 1 n Phosphorsäure 1 Stunde bei 80 °C behandelt. Nach der Behandlung wird mit Wasser gewaschen, bei 110 °C/16 Stunden getrocknet und bei 500 °C/20 Stunden calciniert. Nach einer anderen Arbeitsweise behandelt man Zeolithe vor oder nach ihrer Verformung mit Bindemittel, z.B. 1 bis 3 Stunden bei Temperaturen von 60 bis 80 °C mit einer 3 bis 25 Gew.-%igen, insbesondere 12 bis 20 Gew.-%igen wäßrigen Salzsäure. Anschließend wird der so behandelte Zeolith mit Wasser gewaschen, getrocknet und bei 400 °C bis 500 °C calciniert.

Eine besondere Ausführungsform für die Säurebehandlung besteht darin, daß man das zeolithische

Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen des zeolithischen Materials, wird dieses zweckmäßig, z.B. bei Temperaturen von 100 bis 160° C, getrocknet und bei Temperaturen von im allgemeinen 450° C bis 600° C calciniert. Gemäß einer weiteren bevorzugten Ausführungsform für die Säurebehandlung, wird das zeolithische Material nach einer Verformung mit Bindemittel bei erhöhter Temperatur, zweckmäßig bei Temperaturen von 50 bis 90° C, vorzugsweise 60 bis 80° C, über einen zeitraum von 0,5 bis 5, vorzugsweise mit 12 bis 20 Gew.-%iger Salzsäure, behandelt. Zweckmäßig wird das zeolithische Material anschließend ausgewaschen, bei Temperaturen von 100 bis 160° C getrocknet und bei Temperaturen von 450 bis 600° C calciniert. Einer HF-Behandlung kann sich auch eine HCl-Behandlung anschließen.

Nach einer anderen Arbeitsweise kann man Zeolithe durch Aufbringen von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110° C getrocknet und bei 500° C calciniert.

Die hier beschriebenen Katalysatoren können wahlweise als 2- bis 4 mm-Stränge oder als Tabletten mit 3 bis 5 mm Durchmesser oder als Splitt mit Teilchengrößen von 0,1 bis 0,5 mm oder als Wirbelkontakt eingesetzt werden.

Das Molverhältnis von Lactonen zu $NH_3$ beträgt vorteilhaft von 1:1 bis 1:30, insbesondere 1:3 bis 1:10. Höhere Ammoniakmengen sind möglich, aber unwirtschaftlich. Die Ammoniakzugabe kann gasförmig oder als wäßrige Lösung erfolgen.

Die Umsetzung wird vorteilhaft in der Gasphase durchgeführt, im allgemeinen bei Temperaturen von 100 bis 500° C, vorteilhaft bei 200 bis 450° C, insbesondere 250 bis 400° C. In der Regel führt man die Umsetzung bei einem Druck von 0,1 bis 100 bar, insbesondere 0,5 bis 10 bar durch. Bei der Umsetzung in der Gasphase hält man zweckmäßig eine Katalysatorbelastung von 0,1 bis 20, insbesondere von 1 bis 10 g Lacton je g Katalysator und Stunde ein.

Die Reaktion kann im Festbett oder im Wirbelbett ausgeführt werden. Aber es ist auch möglich, die Reaktion in der Flüssigphase (Suspension-, Riesel- oder Sumpffahrweise) bei Temperaturen zwischen 50 und 220° C durchzuführen. Die Reaktion kann diskontinuierlich, vorzugsweise kontinuierlich erfolgen.

Schwerflüchtige oder feste Ausgangsstoffe kann man in gelöster Form, z.B. in THF-, Toluol- oder Petrolether-Lösung einsetzen. Im allgemeinen ist eine Verdünnung der Ausgangsstoffe mit Lösungsmitteln oder mit Inertgasen wie $N_2$, Ar, $H_2O$-Dampf möglich.

Nach der Umsetzung werden die entstandenen Produkte durch übliche Verfahren, z.B. durch Destillation aus dem Reaktionsgemisch isoliert; nichtumgesetztes Einsatzgemisch wird gegebenenfalls in die Umsetzung zurückgeführt.

Die nach dem erfindungsgemäßen Verfahren erhaltenen ungesättigten Nitrile sind vielseitig verwendbare Zwischenprodukte. Sie eignen sich z.B. für die Herstellung von Faservorprodukten, Pflanzenschutzmitteln und Pharmazeutika.

Beispiele 1 bis 8

Die Reaktion wird in der Gasphase unter isothermen Bedingungen in einem Rohrreaktor (Wendel, 0,6 cm Innendurchmesser, 90 cm Länge) im Verlauf von mindestens 6 Stunden durchgeführt. Das Reaktionsprodukt wird durch übliche Methoden abgetrennt und bestimmt. Die quantitative Bestimmung der Reaktionsprodukte und der nichtumgesetzten Ausgangsstoffe erfolgt gaschromatographisch.

Die für die Beispiele eingesetzten Katalysatoren sind:

Katalysator A

Ein Borosilikatzeolith des Pentasil-Typs wird durch hydrothermale Synthese aus 640 g hochdispersem $SiO_2$, 122 g $H_3BO_3$, 8000 g einer wäßrigen 1,6-Hexandiamin-Lösung (Mischung 50 : 50 Gew.%) bei 170° C unter autogenem Druck in einem Rührautoklaven hergestellt. Nach Abfiltrieren und Auswaschen wird das kristalline Reaktionsprodukt bei 100° C/24 h getrocknet und bei 500° C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit einem Verformungshilfsmittel 2 mm-Stränge hergestellt, die bei 110° C/16 h getrocknet und bei 500° C/24 h calciniert werden.

Katalysator B

Ein Aluminosilikatzeolith vom Pentasil-Typ wird unter hydrothermalen Bedingungen bei autogenem Druck und 150 °C aus 65 g hochdispersem $SiO_2$, 20,3 g $Al_2(SO_4)_3$ x 18 $H_2O$ in 1 kg einer wäßrigen 1,6-Hexandiamin-Lösung (Mischung 50:50 Gew.%) in einem Rührautoklaven hergestellt. Nach Abfiltrieren und Auswaschen wird das kristalline Reaktionsprodukt bei 110 °C/24 h getrocknet und bei 500 °C/24 h calciniert. Dieser Aluminosilikatzeolith enthält 91,6 Gew.% $SiO_2$ und 4,6 Gew.% $Al_2O_3$. Der Zeolith wird mit Boehmit im Gewichtsverhältnis 60:40 zu 2 mm-Strängen verformt, bei 110 °C/16 h getrocknet und bei 500 °C/24 h calciniert.

Katalysator C

Katalysator C erhält man, indem man Katalysator B mit einer wäßrigen $Cr(NO_3)_3$-Lösung imprägniert. Die imprägnierten Stränge werden bei 130 °C/2 h getrocknet und bein 540 °C/2 h calciniert. Der Cr-Gehalt beträgt 2,3 Gew.%.

Katalysator D

Ein handelsüblicher NaY-Zeolith wird mit Beohmit im Gewichtswerhältnis 60:40 verstrangt, bei 110 °C getrocknet und bei 500 °C/16 calciniert und mit 20 %iger Ammoniumchloridlösung einem Ionenaustausch unterworfen. Der Restnatriumgehalt des Katalystors D beträgt 0,2 Gew.% (500 °C calciniert).

Katalysator E

Im Handel erhältlicher Ferrierit (Zeolon 700®) wird mit Boehmit im Gewichtsverhältnis 60:40 zu 2 mm Strängen verformt, danach bei 110 °C getrocknet und bein 500 °C/16 h calciniert. Diese Stränge werden mit 20 %iger Ammoniumchloridlösung einem Ionenaustausch solange unterworfen, bis der Restnatriumgehalt des Katalysators E 0,85 Gew.% beträgt (500 °C/5 h calciniert).

Katalysator F

Borosilikatzeolith von Katalysator A mit Boehmit im Gewichtsverhältnis 60:40 zu 2 mm-Strängen verformt , bei 110 °C/16 h getrocknet und bei 500 °C/24 h calciniert.

Katalysator G

100 g des bei Katalysator A verwendeten Borosilikatzeolithen werden mit 280 ml einer 0,1 n HF bei 90 °C 2 h lang behandelt und nach Abfiltrieren bei 160 °C getrocknet. Dieses Produkt wird mit amorphem Aluminosilikat (25 Gew.% $Al_2O_3$ und 75 Gew.% $SiO_2$) im Gewichtsverhältnis 60:40 zu 2 mm-Strängen verformt, bei 110 °C/16 h getrocknet und bei 500 °C/16 h calciniert.

Die mit diesen Katalysatoren erzielten Versuchsergebnisse und die Reaktionsbedingungen sind in Tabelle 1 und 2 zusammengestellt.

Tabelle 1: Caprolacton (I) + $NH_3$ ⟶ Hexensäurenitrile

| Beispiel | 1 1) | 2 1) | 3 1) | 4 2) | 5 2) |
|---|---|---|---|---|---|
| Katalysator | A | B | C | D | E |
| Temperatur | 400°C | 400°C | 400°C | 400°C | 400°C |
| WHSV | 2,1 $h^{-1}$ | 2,6 $h^{-1}$ | 2,6 $h^{-1}$ | 4,5 $h^{-1}$ | 4,5 $h^{-1}$ |
| I : $NH_3$ molar | 1:7 | 1:7 | 1:7 | 1:1,6 | 1:1,6 |
| Umsatz % | 100 | 100 | 100 | 99 | 100 |
| Selektivität % | | | | | |
| Hexennitrile | 86,1 | 81,5 | 85,7 | 75,0 | 75,2 |

1) $NH_3$ wird als 25 %ige wäßrige Lösung mit Caprolacton gemischt
2) $NH_3$ wird gasförmig getrennt von Caprolacton zugegeben

Nebenprodukte der Reaktion sind Hexan, Hexadiennitrile, Hydroxycapronsäurenitril, Hexensäureamid und in geringem Maße auch Caprolactam.

Tabelle 2

gamma-Valerolacton (I) + $NH_3$ ⟶ Pentensäurenitrile

| Beispiele | 6 | 7 | 8 |
|---|---|---|---|
| Katalysator | F | B | G |
| Temperatur | 400°C | 400°C | 400°C |
| WHSV | 3,5 $h^{-1}$ | 3,5 $h^{-1}$ | 3,5 $h^{-1}$ |
| I : $NH_3$ | 1 : 3 | 1 : 3 | 1 : 3 |
| Umsatz % | 74,7 | 90,9 | 81,7 |
| Selektivität % | | | |
| Butennitrile | 85,0 | 91,6 | 83,5 |

## Patentansprüche

1. Verfahren zur Herstellung Von ungesättigten Nitrilen durch katalytische Spaltung von unsubstituierten oder Alkyl-, Aryl- oder Aralkyl-substituierten 5- bis 7-gliedrigen Lactonen mit Ammoniak, dadurch gekennzeichnet, daß man die Umsetzung im Molverhältnis von Lacton zu Ammoniak von 1 : 1 bis 1 : 30 bei Drücken von 0,1 bis 100 bar in der Gasphase bei 100 bis 500° C oder in der Flüssigphase bei 50 bis 220° C in Gegenwart von Zeolithen als Katalysatoren durchführt.

2. Verfahren nach Anspruch 1, dadurch gekennzeichnet, daß man Caprolacton einsetzt.

3. Verfahren nach den Ansprüchen 1 und 2, dadurch gekennzeichnet, daß man als Katalysatoren Zeolithe des Pentasiltyps verwendet.

4. Verfahren nach den Ansprüchen 1 bis 2, dadurch gekennzeichnet, daß man als Katalysatoren Aluminiumsilikatzeolithe des Faujasittyps verwendet.

5. Verfahren nach den Ansprüchen 1 bis 2, dadurch gekennzeichnet, daß man als Katalysatoren Aluminiumsilikatzeolithe des Erionit- und Chabazittyps verwendet.

6. Verfahren nach den Ansprüchen 1 bis 2, dadurch gekennzeichnet, daß man als Katalysatoren Aluminiumsilikatzeolithe des Ferrierittyps einsetzt.

7. Verfahren nach den Ansprüchen 1 bis 6, dadurch gekennzeichnet, daß man als Katalysatoren mit Alkalimetallen und/oder Erdalkalimetallen und/oder Übergangsmetallen und/oder mit Seltenen Erden dotierte Zeolithe verwendet.

8. Verfahren nach den Ansprüchen 1 bis 7, dadurch gekennzeichnet, daß man die Umsetzung in der Gasphase durchführt.

**Claims**

1. A process for the preparation of an unsaturated nitrile by catalytic cleavage of an unsubstituted or alkyl, aryl- or aralkyl-substituted 5- to 7-membered lactone with ammonia, wherein the reaction is carried out using a molar ratio of lactone to ammonia of from 1 : 1 to 1 : 30 at from 0.1 to 100 bar, at from 100 to 500 °C in the gas phase or at from 50 to 220 °C in the liquid phase, in the presence of a zeolite as a catalyst.

2. A process as claimed in claim 1, wherein caprolactone is used.

3. A process as claimed in claim 1 or 2, wherein the catalyst used is a zeolite of the pentasil type.

4. A process as claimed in claim 1 or 2, wherein the catalyst used is an aluminosilicate zeolite of the faujasite type.

5. A process as claimed in claim 1 or 2, wherein the catalyst used is an aluminosilicate zeolite of the erionite or chabazite type.

6. A process as claimed in claim 1 or 2, wherein the catalyst used is an aluminosilicate zeolite of the ferrierite type.

7. A process as claimed in any of claims 1 to 6, wherein the catalyst used is a zeolite doped with alkali metals and/or alkaline earth metals and/or transition metals and/or rare earths.

8. A process as claimed in any of claims 1 to 7, wherein the reaction is carried out in the gas phase.

**Revendications**

1. Procédé de préparation de nitriles insaturés par ouverture catalytique par l'ammoniac de lactones penta-à heptagonales non substituées ou alkyl-, aryl- ou aralkyl-substituées, caractérisé en ce qu'on conduit la réaction avec un rapport molaire de la lactone à l'ammoniac de 1 : 1 à 1 : 30, sous des pressions de 0,1 à 100 bar en phase gazeuse à 100-500 °C ou en phase liquide à 50-200 °C, en présence de zéolithes servant de catalyseurs.

2. Procédé selon la revendication 1, caractérisé en ce qu'on met en réaction de la caprolactone.

3. Procédé selon la revendication 1 ou 2, caractérisé en ce qu'on utilise, comme catalyseurs, des zéolithes du type pentasil.

4. Procédé selon la revendication 1 ou 2, caractérisé en ce qu'on utilise, comme catalyseurs, des zéolithes d'aluminosilicate du type faujasite.

5. Procédé selon la revendication 1 ou 2, caractérisé en ce qu'on utilise, comme catalyseurs, des zéolithes d'aluminosilicate des types érionite et chabazite.

6. Procédé selon la revendication 1 ou 2, caractérisé en ce qu'on utilise, comme catalyseurs, des zéolithes d'aluminosilicate du type ferriérite.

7. Procédé selon l'une quelconque des revendications 1 à 6, caractérisé en ce qu'on utilise, comme catalyseurs, des zéolithes dopées avec des métaux alcalins et/ou des métaux alcalino-terreux et/ou des métaux de transition et/ou des terres rares.

8. Procédé selon l'une quelconque des revendications 1 à 7, caractérisé en ce qu'on conduit la réaction en phase gazeuse.